# FASCICULE DE BREVET EUROPEEN

(11) **EP 4 208 297 B1**
(45) Date de publication et mention de la délivrance du brevet: **06.11.2024**
(21) Numéro de dépôt: 21778168.1
(22) Date de dépôt: 01.09.2021
(51) Int. Cl.: B05B 11/10, A61M 11/00, A61M 15/00, A61M 15/08, B05B 11/00, B65D 83/26, B65D 83/38

(54) **DISPOSITIF DE DISTRIBUTION DE PRODUIT FLUIDE**
VORRICHTUNG ZUR ABGABE EINES FLÜSSIGEN PRODUKTS
DEVICE FOR DISPENSING A FLUID PRODUCT

(30) Priorité: 01.09.2020 FR 2008864
(43) Date de publication de la demande: 12.07.2023
(73) Titulaire: APTAR France SAS, 27110 Le Neubourg (FR)
(72) Inventeur: FABIEN, David, 29290 Saint Renan (FR)
(74) Mandataire: CAPRI
(86) Numéro de dépôt international: PCT/FR2021/051502
(87) Numéro de publication internationale: WO 2022/049341

(56) Documents cités:
- JP-A- 2011 050 535
- JP-A- S49 109 924
- US-A- 3 952 916
- US-A1- 2002 130 146

## Description

La présente invention concerne un dispositif de distribution de produit fluide.

De nos jours, l'administration de médicaments puissants potentiellement létaux chez l'homme peut-être une nécessité dans certaines situations. C'est notamment le cas pour le traitement de pathologies particulières ou encore pour des personnes ayant besoin de traitements palliatifs dans des contextes de fin de vie. La manipulation de telles substances requiert une grande prudence et des dispositifs d'administration extrêmement sûrs, pour éviter les risques de surdose, qui peuvent survenir en cas d'administration rapprochée de plusieurs doses consécutives. Un autre risque concerne l'utilisation de ces dispositifs par une personne autre que la personne à qui le traitement est destiné, par exemple des enfants.

Par ailleurs, en particulier lorsque le dispositif comporte une pompe actionnée manuellement, l'actionnement du dispositif et la qualité du spray obtenu sont généralement dépendants de la vitesse d'actionnement, et donc fortement dépendants de l'utilisateur. Ainsi, une personne âgée, handicapée ou un enfant est susceptible d'actionner le dispositif avec moins de force et moins vite qu'un adulte valide. De plus, cette dépendance de l'utilisateur résulte en une faible reproductibilité du spray d'une utilisation à l'autre. Ceci peut résulter en une la pénétration des molécules de médicament dans le système ORL plus ou moins efficace suivant l'utilisateur et/ou l'actionnement, avec potentiellement un effet thérapeutique variable d'une utilisation à l'autre.

Les documents JP2011050535, US2002130146, US3952916 et JPS49109924 décrivent des dispositifs de l'état de la technique.

La présente invention a pour but de fournir un dispositif de distribution de produit fluide qui ne reproduit pas les inconvénients susmentionnés.

La présente invention a notamment pour but de fournir un dispositif de distribution de produit fluide qui soit sécurisé et sécurisant pour l'utilisateur, notamment pour éviter les risques d'overdose.

La présente invention a aussi pour but de fournir un dispositif de distribution de produit fluide qui ne soit utilisable que par les personnes autorisées, en particulier par la personne en ayant le besoin thérapeutique.

La présente invention a également pour but de fournir un dispositif de distribution de produit fluide qui soit bloqué pendant un temps prédéterminable entre deux actionnements successifs.

La présente invention a également pour but de fournir un dispositif de distribution de produit fluide qui permette de garantir un spray identique à chaque actionnement, indépendant de l'utilisateur.

La présente invention a aussi pour but de fournir un dispositif de distribution de produit fluide qui soit robuste et fiable d'utilisation.

La présente invention a également pour but de fournir un dispositif de distribution de produit fluide qui soit simple et peu coûteux à fabriquer et à assembler.

La présente invention a donc pour objet un dispositif de distribution de produit fluide comportant:
- un distributeur de produit fluide comportant un réservoir contenant du produit fluide, une tête de distribution pourvue d'un orifice de distribution, ladite tête de distribution étant axialement déplaçable par rapport audit réservoir, et un organe de distribution, tel qu'une pompe ou une valve, monté sur ledit réservoir, ledit organe de distribution étant actionné lorsque ledit réservoir est déplacé axialement vers le haut par rapport à ladite tête de distribution,
- un corps interne comportant un cylindre creux recevant ledit distributeur,
- un corps externe recevant ledit corps interne, un organe de poussée et un système d'actionnement automatique,
- ledit organe de poussée étant en contact avec ledit réservoir et monté axialement mobile par rapport audit corps interne entre une position de repos et une position d'actionnement, un ressort étant interposé entre ledit corps interne et ledit organe de poussée, ledit ressort étant comprimé en position de repos dudit organe de poussée pour le solliciter vers sa position d'actionnement,
- ledit système d'actionnement automatique comportant un engrenage de réarmement, un cliquet, une roue flottante, une roue de moteur et un moteur, dans lequel:
   - ledit cliquet coopère avec ledit organe de poussée pour le bloquer en position de repos, ledit cliquet étant déplacé de sa position de blocage vers une position de libération par ladite roue flottante lorsque le moteur tourne dans un premier sens d'actionnement, et
   - ledit engrenage de réarmement coopère avec ledit organe de poussée, ledit engrenage de réarmement étant déplacé par ladite roue flottante lorsque le moteur tourne dans un second sens de réarmement pour ramener ledit organe de poussée dans sa position de repos.

Avantageusement, la rotation dudit moteur dans ledit premier sens d'actionnement déplace ladite roue flottante pour coopérer avec ledit cliquet, et la rotation dudit moteur dans ledit second sens de réarmement déplace ladite roue flottante pour coopérer avec ledit engrenage de réarmement.

Avantageusement, ledit engrenage de réarmement est monté pivotant sur ledit corps externe et comporte une première denture coopérant avec une denture dudit organe de poussée, et une seconde denture coopérant avec ladite roue flottante lorsque ledit moteur tourne dans ledit second sens de réarmement.

Avantageusement, ledit cliquet est monté pivotant sur ledit corps externe et comporte une dent coopérant avec une encoche dudit organe de poussée.

Avantageusement, une roue de cliquet est montée rotative sur ledit corps externe autour d'un axe de rotation, ledit cliquet comportant une ouverture oblongue montée autour dudit axe de rotation, ladite roue de cliquet coopérant avec ladite roue flottante lorsque ledit moteur tourne dans ledit premier sens d'actionnement.

Avantageusement, ledit cliquet est sollicité vers sa position de blocage, notamment par un élément élastique tel qu'un ressort ou une lame élastique.

Avantageusement, ladite roue flottante comporte un axe de rotation monté mobile dans une ouverture oblongue dudit corps externe.

Avantageusement, ladite roue de moteur est montée rotative sur ledit corps externe et coopère avec ladite roue flottante quand ledit moteur tourne dans ledit premier sens d'actionnement ou dans ledit second sens d'actionnement.

Avantageusement, ledit moteur fait tourner une vis sans fin qui coopère avec ladite roue de moteur.

Avantageusement, le dispositif comporte un module de commande pour commander ledit moteur.

Avantageusement, ledit module de commande comporte des moyens de temporisation pour empêcher, après chaque actionnement du distributeur, le prochain actionnement dudit moteur pendant un temps prédéterminable.

Avantageusement, lesdits moyens de temporisation bloquent un bouton de commande du dispositif et/ou ledit moteur.

Avantageusement, ledit ressort est disposé dans ledit corps interne, entre un fond dudit corps interne et une bride radiale supérieure dudit organe de poussée.

Ces caractéristiques et avantages et d'autres de la présente invention apparaîtront plus clairement au cours de la description détaillée suivante, faite en référence aux dessins joints, donnés à titre d'exemples non limitatifs, et sur lesquels :
la figure 1 est une vue schématique en perspective découpée d'un dispositif de distribution selon un mode de réalisation avantageux,
la figure 2 est une vue schématique partielle du dispositif de la figure 1,
les figures 3 et 4 sont des vues schématiques en perspective éclatée illustrant l'assemblage du distributeur de produit fluide dans le dispositif,
la figure 5a est une vue schématique en section du dispositif de la figure 1, en position de repos,
la figure 5b est une vue schématique de détail agrandie du mécanisme d'actionnement du dispositif de la figure 5a,
les figures 6a et 6b sont des vues similaires à celles des figures 5a et 5b, en début de course d'actionnement,
les figures 7a et 7b sont des vues similaires à celles des figures 6a et 6b, en cours de course d'actionnement,
les figures 8a et 8b sont des vues similaires à celles des figures 7a et 7b, en fin de course d'actionnement,
les figures 9a et 9b sont des vues similaires à celles des figures 8a et 8b, au moment de la distribution d'une dose de produit fluide,
les figures 10a et 10b sont des vues similaires à celles des figures 9a et 9b, en début de course de retour,
les figures 11a et 11b sont des vues similaires à celles des figures 10a et 10b, en fin de course de retour, et
les figures 12a et 12b sont des vues similaires à celles des figures 11a et 11b, de retour en position de repos.

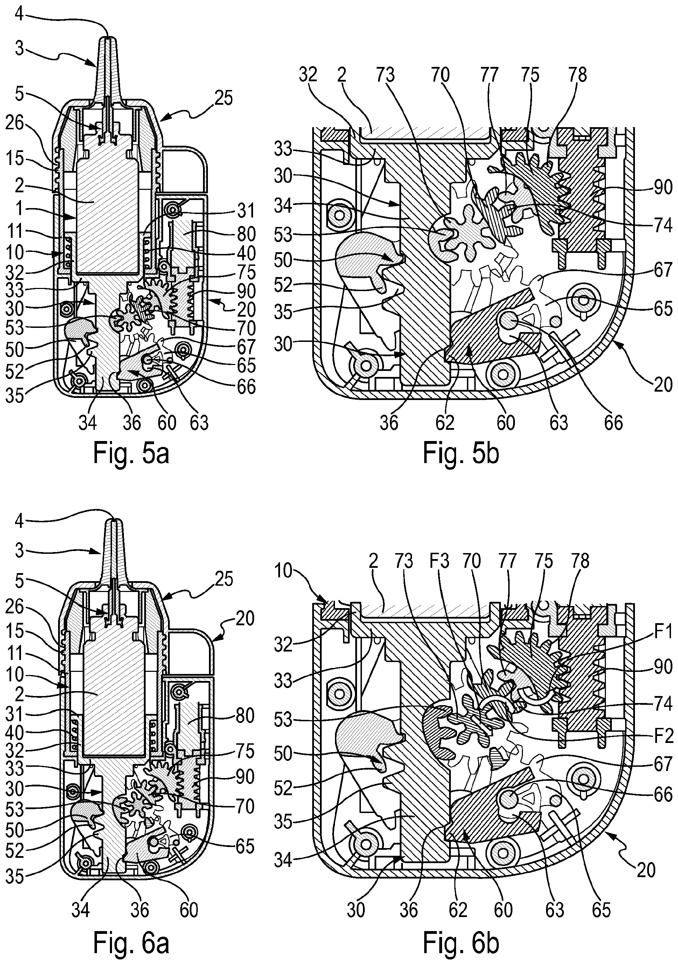

Les termes "axial" et "radial" se réfèrent à l'axe central longitudinal du dispositif. Les termes "haut", "bas", "supérieur" et "inférieur" se réfèrent à la position droite du dispositif représentée notamment sur la figure 5a.

La présente invention a notamment pour objet un dispositif qui autorise/interdit la délivrance de dose en bloquant/libérant l'actionnement d'un distributeur de produit fluide, tout en garantissant un spray identique à chaque actionnement.

Le dispositif de distribution de produit fluide représenté sur les figures comporte un distributeur de produit fluide 1, avantageusement du type standard. Ce distributeur 1 comporte un réservoir 2 contenant le produit fluide et une tête de distribution 3 axialement déplaçable par rapport au réservoir 2. La tête de distribution 3 est pourvue d'un orifice de distribution 4. Un organe de distribution 5, tel qu'une pompe ou une valve, est monté sur le réservoir 2, ledit organe de distribution étant actionné lorsque le réservoir 2 est déplacé axialement vers le haut par rapport à la tête de distribution 3. Généralement, lorsque le réservoir 2 ne contient pas de gaz propulseur, on utilise une pompe doseuse, et lorsque le réservoir 2 contient du gaz propulseur, on utilise une valve doseuse. Ces deux types d'organe de distribution sont bien connus de l'homme du métier et puisque cet organe de distribution n'intervient pas directement dans la présente invention, il ne sera pas décrit plus en détail ci-après. La présente invention est toutefois particulièrement adaptée à l'utilisation d'une pompe sans gaz propulseur.

Le dispositif de distribution de produit fluide comporte également un corps interne 10, recevant le distributeur 1, et un corps externe 20, recevant le corps interne 10.

Le corps interne 10 comporte un cylindre creux 11 ouvert axialement des deux côtés, avec des moyens d'accouplement, avantageusement réalisés sous la forme d'un filetage 15 sur la surface extérieure de l'ouverture supérieur.

Le corps externe 20 est creux et peut avoir une forme extérieure quelconque. Le corps externe 20 se fixe d'une manière appropriée sur le corps interne 10, par exemple par encliquetage. Avantageusement, le corps externe 20 comporte à l'extérieur un ou plusieurs afficheurs (non représentés), par exemple un écran, permettant d'afficher des informations, comme par exemple des instructions d'utilisation, des informations de charge de batterie, etc. L'afficheur peut aussi intégrer une zone d'actionnement, tel qu'un bouton de commande tactile, sur laquelle l'utilisateur appuie pour déverrouiller le dispositif. En variante, un bouton de commande séparé de l'afficheur pourrait aussi être prévu. Avantageusement, le bouton ou la zone de commande peut intégrer des moyens de détection d'empreinte digitale pour n'autoriser l'actionnement du dispositif qu'à la ou aux personnes autorisées, et empêcher ainsi tout actionnement accidentel, par exemple par des enfants. D'autres moyens de reconnaissance pourraient être envisagés, tels qu'une reconnaissance faciale.

Un capot 25 est prévu pour fixer le distributeur 1 dans l'unité formée du corps interne 1 et du corps externe 20. Ce capot 25 comporte un cylindre creux ouvert axialement des deux côtés, avec des moyens d'accouplement complémentaires, avantageusement réalisés sous la forme d'un filetage interne 26, adaptés à coopérer avec les moyens d'accouplement du corps interne 10.

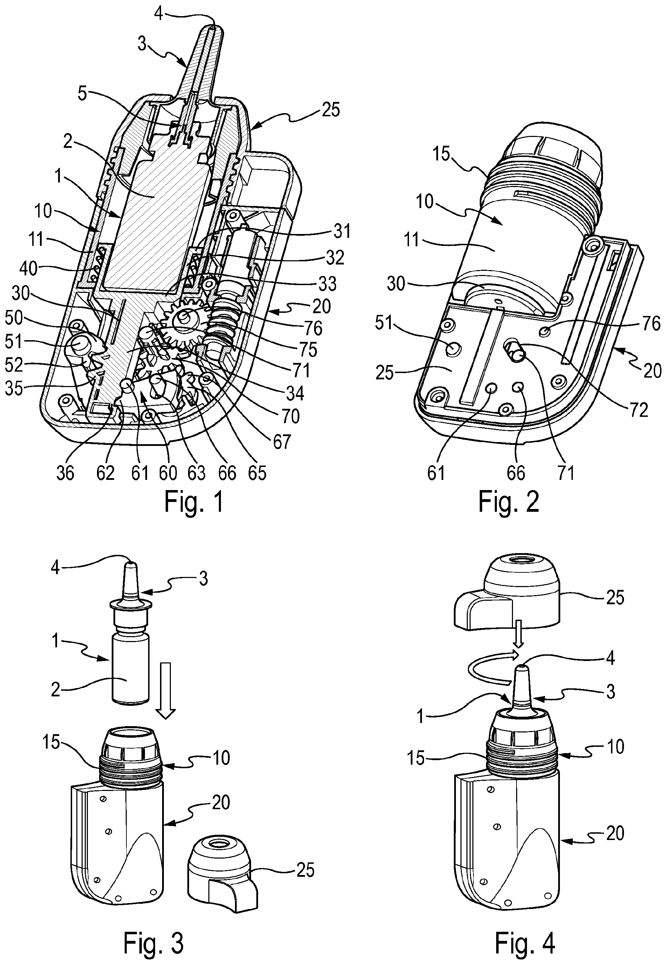

Ainsi, pour assembler le distributeur 1 dans le dispositif, on insère le réservoir 2 à l'intérieur du cylindre creux 11 du corps interne 10. Le capot 25 est alors vissé sur ledit corps interne 10. Avantageusement, lors de ce vissage, une bride radiale de la tête de distribution est coincée entre le bord supérieur du corps interne 10 et le capot 25. Ceci est avantageux en limitant le jeu axial du distributeur 1 dans le dispositif, susceptible de générer des dysfonctionnements. Cet assemblage est représenté sur les figures 3 et 4.

Le dispositif comporte en outre un organe de poussée 30 et un système d'actionnement automatique.

L'organe de poussée 30 se déplace axialement par rapport au corps interne 10 entre une position de repos et une position d'actionnement. Un ressort 40 est disposé dans le corps interne 10, entre un fond dudit corps interne 10 et une bride radiale supérieure 31 de l'organe de poussée 30. Avantageusement, l'organe de poussée 30 est d'abord inséré dans le corps interne 10 par en haut, en étant donc disposé dans ledit corps interne 10 directement sous le réservoir 2.

L'organe de poussée 30 comporte un manchon creux borgne 32 disposé autour du fond du réservoir 2, ledit manchon creux 32 se terminant du côté supérieur par ladite bride radiale supérieure 31 et étant fermé du côté inférieur par une paroi de fond 33. Dans l'exemple représenté, cette paroi de fond 33 traverse l'ouverture inférieure du corps interne 10. Le manchon creux 32 se prolonge axialement vers le bas par une tige axiale 34 comportant une denture 35 et une encoche 36.

Le système d'actionnement automatique comporte un engrenage de réarmement 50, un cliquet 60, une roue de cliquet 65, une roue flottante 70, une roue de moteur 75, une vis sans fin 90 et un moteur 80.

L'engrenage de réarmement 50 est monté pivotant sur le corps externe 20 autour d'un premier axe de pivotement 51, entre une position réarmée et un position actionnée. Il comporte une première denture 52 coopérant avec la denture 35 de la tige axiale 34 de l'organe de poussée 30. L'engrenage de réarmement 50 comporte également une seconde denture 53, dont la fonction sera décrite ultérieurement.

Le cliquet 60 est monté pivotant sur le corps externe 20 autour d'un second axe de pivotement 61, entre une position de blocage et une position de libération. Le cliquet 60 comporte une dent 62 coopérant avec l'encoche 36 de la tige axiale 34 de l'organe de poussée 30. Le cliquet 60 comporte également une ouverture oblongue 63, dont la fonction est décrite ci-après.

La roue de cliquet 65 est montée rotative sur le corps externe 20 autour d'un premier axe de rotation 66, et comporte une troisième denture 67, dont la fonction sera décrite ultérieurement. L'ouverture oblongue 63 du cliquet 60 est montée autour dudit premier axe de rotation 66, permettant au cliquet 60 de se déplacer entre deux positions d'extrémité par rapport à l'axe de rotation 66 de la roue de cliquet 65.

Il est à noter que la cliquet 60 et la roue de cliquet 65 pourrait être réalisés en une seule pièce monobloc. La construction en deux pièces séparées est avantageuse en ce qu'elle permet de réduire la course de pivotement du cliquet 60, et donc l'encombrement du système d'actionnement automatique.

De préférence, le cliquet 60 est sollicité vers sa position de blocage, par exemple par un élément élastique tel qu'un ressort ou une lame élastique.

La roue flottante 70 comporte un second axe de rotation 71 monté mobile dans une ouverture oblongue 72 du corps externe 20. La figure 2 illustre une plaque 25 fixée dans le corps externe 20, et recevant les axes de pivotement et de rotation susmentionnés. Cette plaque 25 comporte ladite ouverture oblongue 72 recevant le second axe de rotation 71 de la roue flottante 70. La roue flottante 70 comporte en outre une quatrième denture 73 et une cinquième denture 74. La quatrième denture 73 coopère avec la troisième denture 67 de la roue de cliquet 65 lorsque la roue flottante 70 tourne dans un premier sens de rotation, et avec la seconde denture 53 de l'engrenage de réarmement 50 lorsque la roue flottante 70 tourne dans l'autre sens de rotation. La fonction de la cinquième denture 74 est décrite ci-après.

La roue de moteur 75 est montée rotative sur le corps externe 20 autour d'un troisième axe de rotation 76, visible sur la figure 2. La roue de moteur 75 comporte une sixième denture 77 coopérant avec la cinquième denture 74 de la roue flottante 70, et une septième denture 78 dont la fonction est décrite ci-après.

Un module de commande (non représenté) peut être fixé sur l'extérieur dudit corps interne 10 et/ou à l'intérieur dudit corps externe 20. Avantageusement, ce module de commande est fixé sur la plaque 25. Ce module de commande actionne le moteur 80 relié à la vis sans fin 90 coopérant avec ladite septième denture 78 de la roue de moteur 75.

Le moteur 80 peut être un moteur à engrenage 3V à courant continu adapté à faire tourner la vis sans fin 90 dans deux directions opposées. Ce moteur peut être alimenté d'une quelconque manière appropriée, par exemple au moyen de batteries ou d'accumulateurs, rechargeables ou non.

La vis sans fin 90 coopère avec la roue de moteur 75 pour la faire tourner dans le sens d'actionnement en début d'actionnement, et dans le sens de réarmement en fin d'actionnement, après distribution d'une dose.

En variante, on pourrait envisager de supprimer la vis sans fin 90, et de faire coopérer le moteur 80 directement avec la roue de moteur 75.

Une carte électronique (non représentée) peut comporter des éléments électroniques appropriés, tels que notamment un microprocesseur, pour faire fonctionner le dispositif, notamment le moteur 80 et l'afficheur. Avantageusement, la carte électronique comporte aussi au moins un switch ou commutateur (non représenté) pour détecter la position angulaire de la vis sans fin 90. Ce switch ou commutateur permet de détecter le début d'actionnement. Ainsi, si après un temps prédéterminable suivant le déverrouillage du dispositif, l'utilisateur n'actionne pas le dispositif, celui-ci peut automatiquement revenir en position de verrouillage. Ledit au moins un switchs ou commutateurs permet également de détecter et d'enregistrer l'actionnement du dispositif, ces informations pouvant être utilisées pour bloquer le dispositif pendant un temps prédéterminé. Ainsi, la carte électronique peut comporter des moyens de temporisation pour n'autoriser un nouvel actionnement qu'après l'expiration d'un délai prédéterminable. Ces moyens de temporisation peuvent notamment comprendre l'horloge interne du microprocesseur. Eventuellement, il est possible de lui adjoindre un composant horloge en temps réel. Ce blocage temporaire agit de préférence sur la commande du moteur 80, empêchant ainsi celui-ci de tourner pour faire tourner la vis sans fin 90. En variante, c'est le bouton de commande qui peut être désactivé ou bloqué pendant un temps prédéterminable. Avantageusement, seule des personnes autorisées, tels que le personnel médical, peuvent modifier ledit temps de blocage en ayant accès à la carte électronique ou via l'afficheur. Avantageusement, l'afficheur indique combien de temps il reste avant de pouvoir et/ou devoir prendre la prochaine dose. Eventuellement, un signal sonore et/ou visuel peut également être prévu si l'utilisateur appuie tout de même sur le bouton de commande pour tenter de déverrouiller le dispositif.

Le fonctionnement du dispositif représenté sur les dessins va maintenant être décrit plus en détail.

Dans un cycle d'actionnement normal, l'utilisateur prend le dispositif dans sa main en position de repos, représentée sur les figures 5a et 5b. Dans cette position de repos, le ressort 40 est comprimé et sollicite donc l'organe de poussée 30 axialement vers le haut, mais ce déplacement axial est bloqué par le cliquet 60, dont la dent 62 est disposée dans l'encoche 36 de l'organe de poussée 30.

Pour actionner le dispositif, l'utilisateur doit réaliser une commande du module de commande, pour faire fonctionner le moteur 80. Pour ce faire, il appuie sur un bouton de commande, qui peut avantageusement être intégré dans un afficheur, ou en variante être prévu sur le corps externe 20. Cet appui va faire tourner le moteur 80 et donc la vis sans fin 90 dans un premier sens d'actionnement.

La rotation de la vis sans fin 90 dans le sens d'actionnement fait tourner la roue de moteur 75 dans le sens d'actionnement, comme illustré par la flèche F1 sur la figure 6b.

Ceci à son tour fait tourner la roue flottante 70 dans le sens d'actionnement, selon la flèche F2 visible sur la figure 6b. L'axe de rotation 71 de la roue flottante étant monté déplaçable dans l'ouverture oblongue 72, cette rotation dans le sens d'actionnement F2 déplace la roue flottante 70 dans l'ouverture oblongue 72 selon la flèche F3.

Ce déplacement de la roue flottante 70 dans l'ouverture oblongue 72 désengage la quatrième denture 73 de la roue flottante 70 de la seconde denture 53 de l'engrenage de poussée 50, et engage cette quatrième denture 73 avec la troisième denture 67 de la roue de cliquet 65.

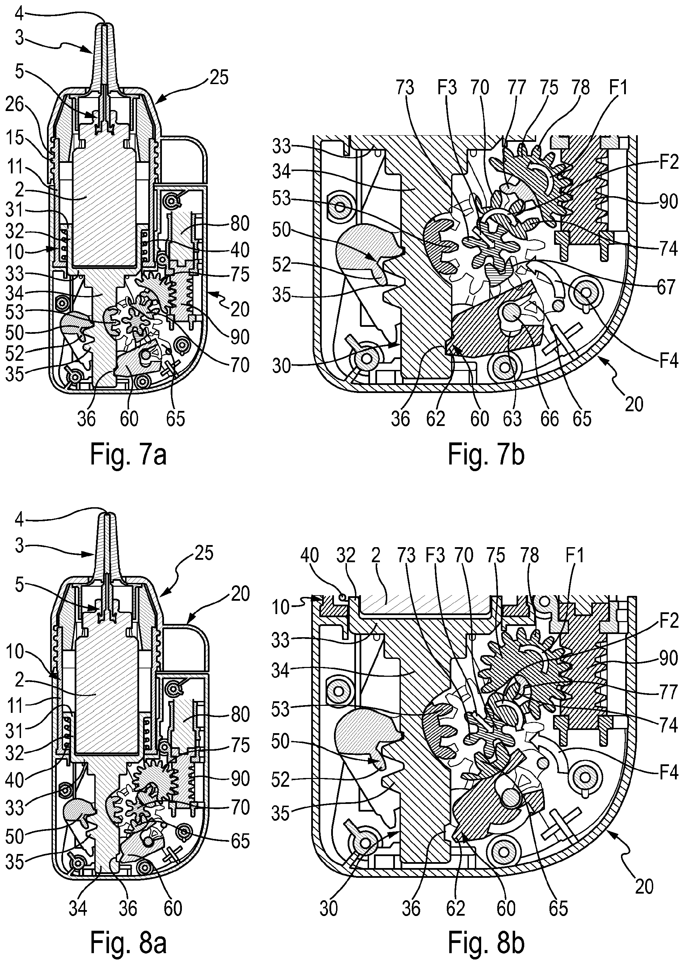

La rotation de la roue flottante 70 dans le sens d'actionnement F2 fait alors tourner la roue de cliquet 65 dans son sens d'actionnement selon la flèche F4 sur la figure 7b.

Cette rotation de la roue de cliquet 65 dans le sens d'actionnement F4 fait pivoter le cliquet 60 autour de son axe de pivotement 61, avec l'axe de rotation 66 de la roue de cliquet 65 qui se déplace dans l'ouverture oblongue 63 du cliquet 60. Ce déplacement du cliquet 60 vers sa position de libération, visible sur la figure 8B, permet de désengager la dent 62 du cliquet 60 de l'encoche 36 de l'organe de poussée 30.

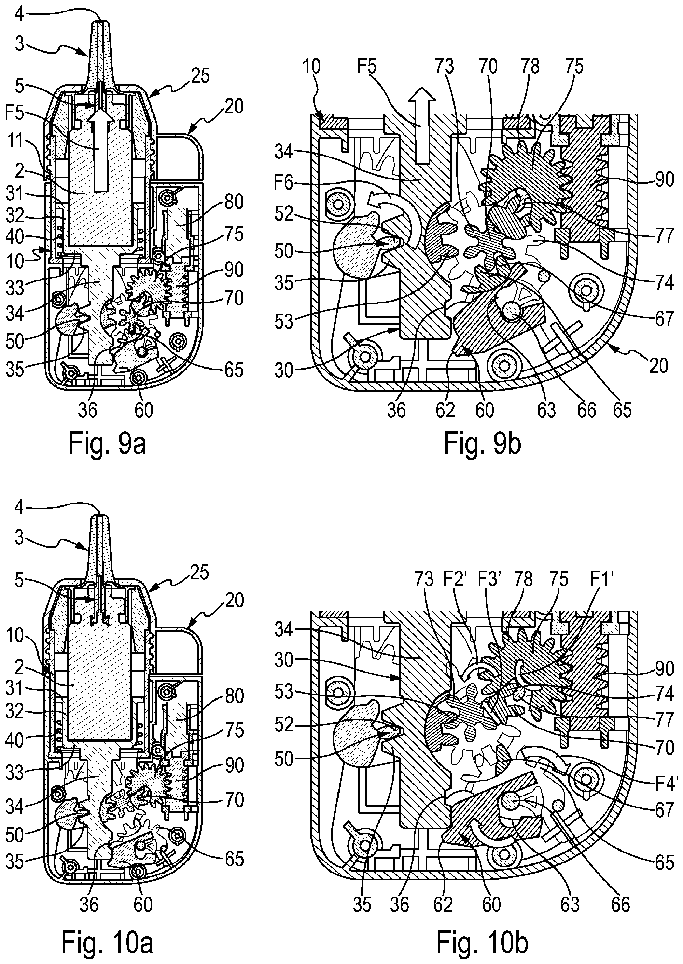

L'organe de poussée 30 est alors automatiquement déplacé axialement vers le haut par le ressort 40, ce qui déplace le réservoir 2, et ainsi actionne l'organe de distribution 5, comme visible sur les figures 9a et 9b. Cet actionnement étant réalisé par le ressort 40, il sera réalisé avec la même force et à la même vitesse à chaque actionnement, ce qui garantit un spray identique à chaque actionnement.

Ce déplacement axial vers le haut de l'organe de poussée 30 entraine la rotation de l'engrenage de réarmement 50 dans un premier sens selon la flèche F6 représentée sur la figure 9b. Cette rotation de l'engrenage de réarmement 50 vers sa position actionnée est possible grâce à l'absence de coopération dans cette position entre la roue flottante 70 et l'engrenage de réarmement 50.

Après distribution de la dose, le système d'actionnement automatique va être réarmé pour une prochaine utilisation.

Le moteur 80 cesse de tourner dans le sens d'actionnement. Avantageusement, c'est le déplacement axial de l'organe de poussée 30 et/ou du réservoir 2 qui déclenche un switch adapté à faire stopper le moteur 80.

Pour le réarmement, le module de commande va faire tourner le moteur 80 et donc la vis sans fin 90 dans un second sens de réarmement, inverse au premier sens d'actionnement.

La rotation de la vis sans fin 90 dans le sens de réarmement fait tourner la roue de moteur 75 dans le sens de réarmement opposé au sens d'actionnement, comme illustré par la flèche F1' sur la figure 10b.

Ceci à son tour fait tourner la roue flottante 70 dans le sens de réarmement selon la flèche F2' visible sur la figure 10b. L'axe de rotation 71 de la roue flottante étant monté déplaçable dans l'ouverture oblongue 72, cette rotation dans le sens d'actionnement F2' déplace la roue flottante 70 dans l'ouverture oblongue 72 selon la flèche F3'.

Ce déplacement de la roue flottante 70 dans l'ouverture oblongue 72 selon la flèche F3' désengage la quatrième denture 73 de la roue flottante 70 de la troisième denture 67 de la roue de cliquet 65, et engage cette quatrième denture 73 avec la seconde denture 53 de l'engrenage de poussée 50.

La roue de cliquet 65 et le cliquet 60 n'étant plus engrenés dans la roue flottante 70, ils peuvent revenir à leur position de départ.

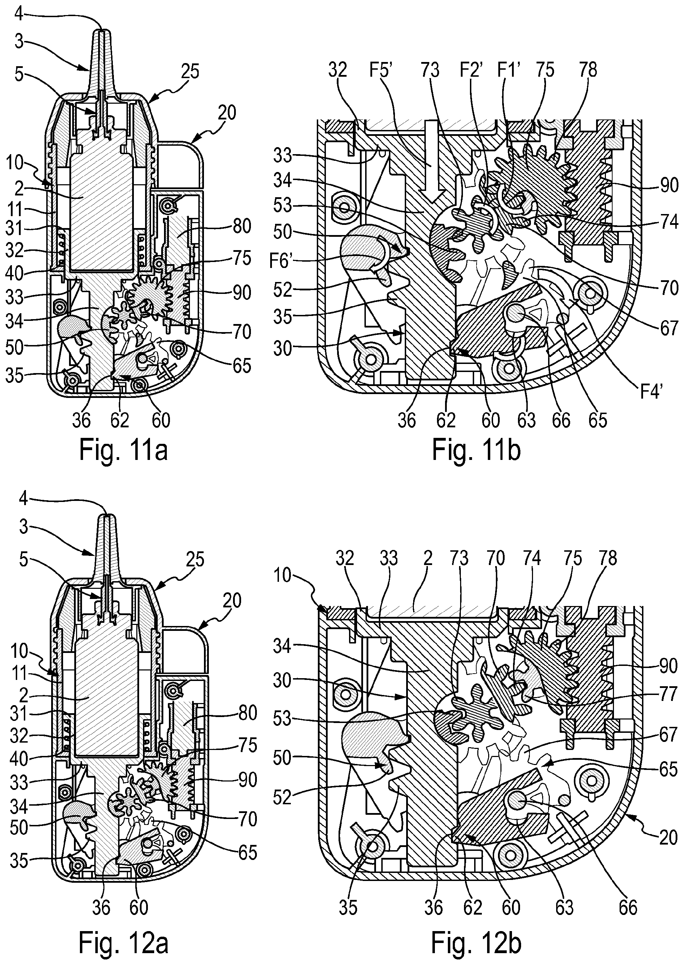

La rotation de la roue flottante 70 dans le sens de réarmement F2' fait alors pivoter l'engrenage de poussée 50 vers sa position réarmée selon la flèche F6' sur la figure 11b.

Cette rotation de l'engrenage de poussée 50 vers sa position réarmée déplace alors l'organe de poussée 30 axialement vers le bas, en comprimant le ressort 40, comme illustré sur les figures 11a et 11b.

Lorsque le dispositif revient en position de repos, visible sur les figures 12a et 12b, la dent 62 du cliquet 60 bloque à nouveau l'organe de poussée 30, et le moteur 80 cesse de tourner dans le sens de réarmement. Avantageusement, c'est le déplacement axial de l'organe de poussée 30 et/ou du réservoir 2 qui déclenche un switch adapté à faire stopper le moteur 80.

Avantageusement, lorsque l'organe de poussée 30 revient en position de repos, le moteur 80 tourne de quelques degrés dans le premier dans d'actionnement, afin de bien positionner le doigt 62 du cliquet 60 dans l'encoche 36 de l'organe de poussée 30, afin de garantir un parfait verrouillage.

Grâce à la transmission du couple du moteur 80 à l'organe de poussée 30 par l'intermédiaire de plusieurs engrenages, on diminue le couple moteur nécessaire, ce qui permet d'utiliser un moteur de faible puissance.

De préférence, un prochain actionnement ne sera possible qu'après l'expiration du délai de blocage prédéterminé par le module de commande.

Bien que la présente invention ait été décrite en référence à un mode de réalisation avantageux, il est entendu que l'homme du métier peut y apporter diverses modifications sans sortir du cadre de la présente invention tel que défini par les revendications annexées.

## Revendications

1. Dispositif de distribution de produit fluide comportant:
- un distributeur de produit fluide (1) comportant un réservoir (2) contenant du produit fluide, une tête de distribution (3) pourvue d'un orifice de distribution (4), ladite tête de distribution (3) étant axialement déplaçable par rapport audit réservoir (2), et un organe de distribution (5), tel qu'une pompe ou une valve, monté sur ledit réservoir (2), ledit organe de distribution (5) étant actionné lorsque ledit réservoir (2) est déplacé axialement vers le haut par rapport à ladite tête de distribution (3),
- un corps interne (10) comportant un cylindre creux (11) recevant ledit distributeur (1),
- un corps externe (20) recevant ledit corps interne (10), un organe de poussée (30) et un système d'actionnement automatique,
- ledit organe de poussée (30) étant en contact avec ledit réservoir (2) et monté axialement mobile par rapport audit corps interne (10) entre une position de repos et une position d'actionnement, un ressort (40) étant interposé entre ledit corps interne (10) et ledit organe de poussée (30), ledit ressort (40) étant comprimé en position de repos dudit organe de poussée (30) pour le solliciter vers sa position d'actionnement,
- ledit système d'actionnement automatique comportant un engrenage de réarmement (50), un cliquet (60), une roue flottante (70), une roue de moteur (75) et un moteur (80), dans lequel:
- ledit cliquet (60) coopère avec ledit organe de poussée (30) pour le bloquer en position de repos, ledit cliquet (60) étant déplacé de sa position de blocage vers une position de libération par ladite roue flottante (70) lorsque le moteur (80) tourne dans un premier sens d'actionnement, et
- ledit engrenage de réarmement (50) coopère avec ledit organe de poussée (30), ledit engrenage de réarmement (50) étant déplacé par ladite roue flottante (70) lorsque le moteur (80) tourne dans un second sens de réarmement pour ramener ledit organe de poussée (30) dans sa position de repos.

2. Dispositif selon la revendication 1, dans lequel la rotation dudit moteur (80) dans ledit premier sens d'actionnement déplace ladite roue flottante (70) pour coopérer avec ledit cliquet (60), et la rotation dudit moteur (80) dans ledit second sens de réarmement déplace ladite roue flottante (70) pour coopérer avec ledit engrenage de réarmement (50).

3. Dispositif selon la revendication 1 ou 2, dans lequel ledit engrenage de réarmement (50) est monté pivotant sur ledit corps externe (20) et comporte une première denture (52) coopérant avec une denture (35) dudit organe de poussée (30), et une seconde denture (53) coopérant avec ladite roue flottante (70) lorsque ledit moteur tourne dans ledit second sens de réarmement.

4. Dispositif selon l'une quelconque des revendications précédentes, dans lequel ledit cliquet (60) est monté pivotant sur ledit corps externe (20) et comporte une dent (62) coopérant avec une encoche (36) dudit organe de poussée (30).

5. Dispositif selon la revendication 4, dans lequel une roue de cliquet (65) est montée rotative sur ledit corps externe (20) autour d'un axe de rotation (66), ledit cliquet (60) comportant une ouverture oblongue (63) montée autour dudit axe de rotation (66), ladite roue de cliquet (65) coopérant avec ladite roue flottante (70) lorsque ledit moteur (80) tourne dans ledit premier sens d'actionnement.

6. Dispositif selon l'une quelconque des revendications précédentes, dans lequel ledit cliquet (60) est sollicité vers sa position de blocage, notamment par un élément élastique tel qu'un ressort ou une lame élastique.

7. Dispositif selon l'une quelconque des revendications précédentes, dans lequel ladite roue flottante (70) comporte un axe de rotation (71) monté mobile dans une ouverture oblongue (72) dudit corps externe (20).

8. Dispositif selon l'une quelconque des revendications précédentes, dans lequel ladite roue de moteur (75) est montée rotative sur ledit corps externe (20) et coopère avec ladite roue flottante (70) quand ledit moteur (80) tourne dans ledit premier sens d'actionnement ou dans ledit second sens d'actionnement.

9. Dispositif selon l'une quelconque des revendications précédentes, dans lequel ledit moteur (80) fait tourner une vis sans fin (90) qui coopère avec ladite roue de moteur (75).

10. Dispositif selon l'une quelconque des revendications précédentes, comportant un module de commande pour commander ledit moteur (80).

11. Dispositif selon la revendication 10, dans lequel ledit module de commande comporte des moyens de temporisation pour empêcher, après chaque actionnement du distributeur (1), le prochain actionnement dudit moteur (80) pendant un temps prédéterminable.

12. Dispositif selon la revendication 11, dans lequel lesdits moyens de temporisation bloquent un bouton de commande du dispositif et/ou ledit moteur (80).

13. Dispositif selon l'une quelconque des revendications précédentes, dans lequel ledit ressort (40) est disposé dans ledit corps interne (10), entre un fond dudit corps interne (10) et une bride radiale supérieure (31) dudit organe de poussée (30).

## Patentansprüche

1. Vorrichtung zur Abgabe eines Fluidprodukts, welche umfasst:
- einen Fluidprodukt-Spender (1), der einen Fluidprodukt enthaltenden Behälter (2), einen mit einer Abgabeöffnung (4) versehenen Abgabekopf (3), wobei der Abgabekopf (3) bezüglich des Behälters (2) axial verschiebbar ist, und ein Abgabeorgan (5) wie etwa eine Pumpe oder ein Ventil, das auf dem Behälter (2) angebracht ist, umfasst, wobei das Abgabeorgan (5) betätigt wird, wenn der Behälter (2) bezüglich des Abgabekopfes (3) axial nach oben verschoben wird,
- einen inneren Körper (10), der einen Hohlzylinder (11) umfasst, welcher den Spender (1) aufnimmt,
- einen äußeren Körper (20), der den inneren Körper (10), ein Schuborgan (30) und ein System zur automatischen Betätigung aufnimmt,
- wobei das Schuborgan (30) mit dem Behälter (2) in Kontakt steht und bezüglich des inneren Körpers (10) axial beweglich zwischen einer Ruheposition und einer Betätigungsposition angebracht ist, wobei eine Feder (40) zwischen dem inneren Körper (10) und dem Schuborgan (30) angeordnet ist, wobei die Feder (40) in der Ruheposition des Schuborgans (30) zusammengedrückt wird, um es in Richtung seiner Betätigungsposition zu beaufschlagen,
- wobei das System zur automatischen Betätigung ein Rückstellgetriebe (50), eine Sperrklinke (60), ein loses Rad (70), ein Motor-Rad (75) und einen Motor (80) umfasst, wobei:
- die Sperrklinke (60) mit dem Schuborgan (30) zusammenwirkt, um es in der Ruheposition zu blockieren, wobei die Sperrklinke (60) durch das lose Rad (70) aus ihrer Blockierposition zu einer Freigabeposition hin bewegt wird, wenn sich der Motor (80) in einer ersten Betätigungsrichtung dreht, und
- wobei das Rückstellgetriebe (50) mit dem Schuborgan (30) zusammenwirkt, wobei das Rückstellgetriebe (50) durch das lose Rad (70) bewegt wird, wenn sich der Motor (80) in einer zweiten Rückstellrichtung dreht, um das Schuborgan (30) in seine Ruheposition zurückzubewegen.

2. Vorrichtung nach Anspruch 1, wobei die Drehung des Motors (80) in der ersten Betätigungsrichtung das lose Rad (70) so bewegt, dass es mit der Sperrklinke (60) zusammenwirkt, und die Drehung des Motors (80) in der zweiten Rückstellrichtung das lose Rad (70) so bewegt, dass es mit dem Rückstellgetriebe (50) zusammenwirkt.

3. Vorrichtung nach Anspruch 1 oder 2, wobei das Rückstellgetriebe (50) schwenkbar am äußeren Körper (20) angebracht ist und eine erste Zahnung (52), die mit einer Zahnung (35) des Schuborgans (30) zusammenwirkt, und eine zweite Zahnung (53), die mit dem losen Rad (70) zusammenwirkt, wenn sich der Motor in der zweiten Rückstellrichtung dreht, umfasst.

4. Vorrichtung nach einem der vorhergehenden Ansprüche, wobei die Sperrklinke (60) schwenkbar am äußeren Körper (20) angebracht ist und einen Zahn (62) umfasst, der mit einer Aussparung (36) des Schuborgans (30) zusammenwirkt.

5. Vorrichtung nach Anspruch 4, wobei ein Klinkenrad (65) um eine Drehachse (66) drehbar am äußeren Körper (20) gelagert ist, wobei die Sperrklinke (60) eine längliche Öffnung (63) umfasst, die um die Drehachse (66) herum angebracht ist, wobei das Klinkenrad (65) mit dem losen Rad (70) zusammenwirkt, wenn sich der Motor (80) in der ersten Betätigungsrichtung dreht.

6. Vorrichtung nach einem der vorhergehenden Ansprüche, wobei die Sperrklinke (60) zu ihrer Blockierposition hin beaufschlagt wird, insbesondere durch ein elastisches Element wie etwa eine Feder oder eine elastische Lamelle.

7. Vorrichtung nach einem der vorhergehenden Ansprüche, wobei das lose Rad (70) eine Drehachse (71) umfasst, die beweglich in einer länglichen Öffnung (72) des äußeren Körpers (20) angebracht ist.

8. Vorrichtung nach einem der vorhergehenden Ansprüche, wobei das Motor-Rad (75) drehbar am äußeren Körper (20) angebracht ist und mit dem losen Rad (70) zusammenwirkt, wenn sich der Motor (80) in der ersten Betätigungsrichtung oder in der zweiten Betätigungsrichtung dreht.

9. Vorrichtung nach einem der vorhergehenden Ansprüche, wobei der Motor (80) eine Schnecke (90) in Rotation versetzt, die mit dem Motor-Rad (75) zusammenwirkt.

10. Vorrichtung nach einem der vorhergehenden Ansprüche, welche ein Steuermodul zur Steuerung des Motors (80) umfasst.

11. Vorrichtung nach Anspruch 10, wobei das Steuermodul Verzögerungsmittel umfasst, um nach jeder Betätigung des Spenders (1) die nächste Betätigung des Motors (80) während einer vorbestimmbaren Zeit zu verhindern.

12. Vorrichtung nach Anspruch 11, wobei die Verzögerungsmittel einen Bedienknopf der Vorrichtung und/oder den Motor (80) blockieren.

13. Vorrichtung nach einem der vorhergehenden Ansprüche, wobei die Feder (40) im inneren Körper (10) zwischen einem Boden des inneren Körpers (10) und einem oberen radialen Flansch (31) des Schuborgans (30) angeordnet ist.

## Claims

1. A device for dispensing a fluid product, comprising:
- a fluid product dispenser (1) comprising: a reservoir (2) containing a fluid product; a dispensing head (3) that is provided with a dispensing orifice (4), said dispensing head (3) being able to move axially relative to said reservoir (2), and a dispensing member (5), such as a pump or a valve, that is mounted on said reservoir (2), said dispensing member (5) being actuated when said reservoir (2) is moved axially upwards relative to said dispensing head (3),
- an inner body (10) comprising a hollow cylinder (11) receiving said dispenser (1),
- an outer body (20) that receives said inner body (10), a push member (30), and an automatic actuating system,
- said push member (30) being in contact with said reservoir (2) and mounted axially movable relative to said inner body (10) between rest position and an actuating position, a spring (40) being interposed between said inner body (10) and said push member (30), said spring (40) being compressed in the rest position of said push member (30) in order to urge it towards its actuating position,
- said automatic actuating system comprising a reset gear (50), a pawl (60), a floating wheel (70), a motor wheel (75), and a motor (80), wherein:
- said pawl (60) interacts with said push member (30) to lock it in the rest position, said pawl (60) being moved from its locked position towards a release position by said floating wheel (70) when the motor (80) rotates in a first actuating direction, and
- said reset gear (50) interacts with said push member (30), said reset gear (50) being moved by said floating wheel (70) when the motor (80) rotates in a second reset direction in order to return said push member (30) into its rest position.

2. The device according to claim 1, wherein rotation of said motor (80) in said first actuating direction moves said floating wheel (70) to interact with said pawl (60), and rotation of said motor (80) in said second reset direction moves said floating wheel (70) to interact with said reset gear (50).

3. The device according to claim 1 or claim 2, wherein said reset gear (50) is pivotally mounted on said outer body (20) and includes a first set of teeth (52) interacting with a set of teeth (35) of said push member (30), and a second set of teeth (53) interacting with said floating wheel (70) when said motor rotates in said second reset direction.

4. The device according to any one of the preceding claims, wherein said pawl (60) is pivotally mounted on said outer body (20) and includes a tooth (62) that interacts with a notch (36) in said push member (30).

5. The device according to claim 4, wherein a pawl wheel (65) is mounted to rotate on said outer body (20) about an axis of rotation (66), said pawl (60) including an oblong opening (63) mounted about said axis of rotation (66), said pawl wheel (65) interacting with said floating wheel (70) when said motor (80) rotates in said first actuating direction.

6. The device according to any one of the preceding claims, wherein said pawl (60) is biased towards its locking position, in particular by a resilient element such as a spring or an resilient blade.

7. The device according to any one of the preceding claims, wherein said floating wheel (70) includes a rotation axis (71) movably mounted in an oblong opening (72) of said outer body (20).

8. The device according to any one of the preceding claims, wherein said motor wheel (75) is rotatably mounted on said outer body (20) and interacts with said floating wheel (70) when said motor (80) rotates in said first actuating direction or in said second actuating direction.

9. The device according to any one of the preceding claims, wherein said motor (80) rotates a worm screw (90) which interacts with said motor wheel (75).

10. The device according to any one of the preceding claims, comprising a control module to control said motor (80).

11. The device according to claim 10, wherein said control module includes timing means for preventing, after each actuation of the dispenser (1), the next actuation of said motor (80) for a predeterminable time.

12. The device according to claim 11, wherein said timing means locks a control button (85) of the device and/or said motor (80).

13. The device according to any one of the preceding claims, wherein said spring (40) is arranged in said inner body (10), between a bottom of said inner body (10) and an upper radial flange (31) of said push member (30).
